# EUROPEAN PATENT SPECIFICATION

(11) **EP 1 218 093 B1**
(45) Date of publication and mention of the grant of the patent: **28.11.2007**
(21) Application number: 00965021.9
(22) Date of filing: 14.09.2000
(51) Int. Cl.: B01D 69/12, B01D 61/18

(54) **VIRUS REMOVAL DEVICES**
VORRICHTUNG ZUR ENTFERNUNG VON VIREN
DISPOSITIF D'ELIMINATION DE VIRUS

(30) Priority: 14.09.1999 US 153830 P
(43) Date of publication of application: 03.07.2002
(73) Proprietor: MILLIPORE CORPORATION, Billerica Massachusetts 01821 (US); Tkacik, Gabriel, Bedford, MA 01730 (US); Kazan, Greg, Danvers, MA 01923 (US)
(72) Inventor: TKACIK, Gabriel, Bedford, MA 01730 (US); KAZAN, Greg, Danvers, MA 01923 (US)
(74) Representative: Henkel, Feiler & Hänzel
(86) International application number: PCT/US2000/025249
(87) International publication number: WO 2001/019506

(56) References cited:
- EP-A- 0 083 489
- EP-A- 0 364 173
- US-A- 4 261 834
- US-A- 4 983 288

## Description

### BACKGROUND OF THE INVENTION

Virus removal from liquid streams, particularly process streams in the biotech and pharmaceutical industry, has been practiced for some time. High viral clearance, high product flux, complete protein passage and simplicity of operation are the goals of the operator, yet the prior art does not provide a solution that satisfies all of these goals. Since high viral clearance is always needed, it is the other process goals that have suffered. Meeting these other goals would substantially lower processing cost.

The prior art provides several membrane types and filtration modes for viral clearance. For a general discussion concerning the state of prior art, see Chapter 20 of Filtration in the Biopharmaceutical Industry, Marcel Dekker, Inc. (1988). Chapter 20 is entitled "Filtration and the Removal of Viruses from Biopharmaceuticals."

One of the products discussed in Filtration was the Viresolve^{™} product line produced by Millipore Corporation. This is a system that used a composite ultrafiltration membrane in a tight side-up-stream (TSUS) orientation in a tangential flow filtration (TFF) device.

U.S. Patent No. 5,017,292 discloses technology used to produce the Viresolve product. It provides a composite membrane comprising a porous membrane substrate, a tight side (the surface having smaller diameter pores) having ultrafiltration separation properties and an intermediate porous zone between the substrate and the skin which intermediate zone has an average pore size smaller than that of the substrate. The intermediate zone is free of macrovoids that may break the skin. The composite membrane is capable of a log reduction value (LRV) of at least 3 (99.9% removal) of virus particles (collectively "virus") selectively from solution. A limitation of this system is that in order to achieve adequate protein solution flux, a complex pumping system is needed to operate effectively in a TFF mode. This needed complexity results in a substantial filtration cost.

While more conventional virus removal applications are available from several manufacturers, they also cannot attain all the goals set forth above. Indeed, they either use conventional ultrafiltration membranes in single layer TSUS orientation in a TFF device or a hollow fiber ultrafiltration TFF device. They similarly lack the simplicity of use and result in a high filtration cost.

Normal flow filtration (NFF) devices, also known as dead - ended filtration devices, are currently available for use in removing viruses from process streams. Indeed, Pall Corp. of East Hills, New York manufactures a dead-ended virus removal membrane under the Ultipore® DV50 brand (hereinafter the "DV50") and DV20 brand (hereinafter the "DV20").

The DV50 consists of three-layers of an isotropic, skinless, porous polyvinylidene fluoride ("PVDF") membrane. While this product has the desired virus removal capabilities and simplicity of use for large virus removal, the isotropic structure of the membranes employed limits its permeability. A low permeability (water permeability of 2 lmh/psi) increases the costs of filtration. This product also does not meet all the goals set forth above.

The DV20 is similar product, but designed for small virus removal. It has a permeability of 0.087 lmh/kPa (0.6 lmh/psi) with IgG..

U.S. Patent No. 5,736,051 discloses a PVDF membrane and method for removing viruses from solutions. More particularly, it provides an isotropic, skinless, porous PVDF membrane. We believe this is the membrane used in the DV50 and DV20.

U.S. Patent No. 5,788,862 discloses a supported ultrafiltration membrane with a coated skin. In the patent, the membrane is described as having been used in a 2-layer configuration in NFF mode with a coated skin to filter viruses from protein streams. While satisfactory levels of virus removal were achieved, the protein passage and flux were very low: maximum disclosed flux with IgG was 0.6 lmh/psi and max IgG passage was only 84%.

In addition to the prior art providing multi-layered virus removal membranes, the prior art also provides multi-layered ultrafiltration membranes with at least one membrane being oriented TSDS. U.S. Patent No. 4,261,834 provides two anisotropic ultrafiltration membranes positioned in series with at least one membrane juxtaposed with at least one other membrane so that substantially all the skin surface of one membrane is in intimate contact with substantially all of the skin surface of the other membrane. This invention, however, was directed to mask pinhole defects for ultrafiltration membranes used to remove proteins (pyrogens) from aqueous streams in a tangential flow filtration (TFF) system.

EP-A-0 083 489 discloses a membrane system useful for filtering a fluid, such as a liquid, which contains particles such as bacteria, to be removed from the fluid. The membrane system comprises at least two members selected from the group consisting of at least one pre-filter and at least one porous asymmetric membrane. The asymmetric membrane has a skin or shiny side and a support or dull side. The membranes are arranged parallel to and in intimate contact with each other such that the fluid passes through each membrane. The reference describes a bubble point of 379.21 kPa (55 psi) for a single layer and of 482.63 kPa (70 psi) for a double layer membrane system.

US-A-5 017 292 discloses an asymmetric membrane and a process for isolating virus from a protein solution. The membrane is a composite membrane having a porous substrate, a surface skin having ultrafiltration properties and an intermediate porous zone between the skin and substrate The skin surface may be rendered hydrophilic if required.

It is the object of the present invention to provide a device for removing virus from a liquid which provides a high flux, has a high virus removal capability and substantially complete protein passage.

### SUMMARY OF THE INVENTION

The present invention provides a device for removing virus from a liquid as defined in claim 1 and a method for removing virus from a liquid using the device of the invention.

Preferred embodiments of the device are defined in the dependent claims.

The present invention provides a membrane suitable for use in a multi-layered, virus removal device, the membrane comprising a tight side, a porous support, and a pore size range suitable for ultrafiltration, the membrane further characterized by having asymmetric structure substantially free of macrovoids (void-free) and having surfaces that if exposed to a protein laden solution, exhibit low protein binding.

The present invention provides a virus removal device comprising filtration material and characterized by having a Vmax greater than 10 ml/cm² and an LRV >6 and protein passage of >98% when challenged with a TSUSpension of at least 10 pfu/ml of bacteriophage φ6 (size 78 nm) and monoclonal IgG at a concentration 2.5 mg/ml up to volumes of 50 ml/cm² of tested filter. Preferably, the Vmax is at least 18 ml/ cm².

The present invention provides a process for producing a polymeric ultrafiltration membrane suitable for virus removal, the process comprising dissolving a polymeric material in a suitable solvent, filtering and degassing the casting solution, heating the casting solution to its cloud point, casting the solution onto a belt passing over a casting drum submerged in water, the solution having a short residence time on the drum; using a knife to set the cast thickness, exposing the cast film to dry air, immersing the cast film in the water bath, extracting the membrane from the water bath, and hydrophilizing the membrane in a monomer solution.

### BRIEF DESCRIPTION OF THE FIGURES

Figure 1 provides a SEM of a layer of membrane as described in Example 1. This layer is representative of a high integrity ultrafiltration membrane.
Figure 2 provides a cross section of a "grenade" type housing used in the device of the present invention.
Figure 3 provides a graph illustrating the performance and protein clearance of a single layer and dual layer virus removal device with composite membrane as the filtration material.

### DETAILED DESCRIPTION OF THE SPECIFIC EMBODIMENT

There is a clear need for filtration materials and devices that remove viruses at high log reduction values (LRV), having essentially complete passage of the protein product and operating at high flux. Furthermore, there is a need that such materials and devices should be easy to operate, preferably in NFF mode.

Unless otherwise defined, terms used in this application are to be construed in accordance with the publication entitled "Terminology for membranes and membrane processes" prepared under the auspices of the INTERNATIONAL UNION OF PURE AND APPLIED CHEMISTRY (IUPAC) and found at
http://www.che.utexas.edu/nams/IUPAC/iupac.html.

The present inventive filtration medium can be used in suitable filters, filtration cartridges, and the like. Of course, in view of the excellent removal efficiency of the present inventive filtration medium, as well as its low susceptibility to protein adsorption, the present inventive filtration medium can be used in dead-end filtration applications, as well as in tangential, cross-flow, and dynamic filtration applications.

For purposes of this invention, a "a high integrity ultrafiltration membrane" is an asymmetric ultrafiltration membrane substantially free of macrovoids and achieving a minimal automated ramp bubble point (APB) of 599.84 kPa (87 psi) when tested in TSDS orientation.

In a preferred embodiment, protein minimally binds to the surfaces of the filtration material of the present invention.

For purposes of this invention, a "pre-filter layer" means any material used to filter matter from the stream of interest with the objective to increase the throughput of the filter device. Such a pre-filter layer would typically have lower viral retention than the upstream layer of the multi-layered ultrafiltration material of the present invention.

For purposes of this invention, a "multi-layered membrane filtration material" includes membranes with a minimal distance between them and includes membranes with a measurable distance between them, including separate housings. The preferred embodiment of the present invention is where the layers of the multi-layered membrane are stacked one on top of the other and contained in the same housing.

The present inventive filtration medium is expected to be especially useful in filter elements, such as filter cartridges, which are known to those of ordinary skill in the art. Preferred filter elements utilizing the present inventive filtration medium comprise the present inventive filtration medium in sheet form, wherein the layers of membrane are stacked one upon the other and are bonded with a thermoplastic seal inside a housing, such as the Millex^{™} filter cartridges sold by Millipore Corporation.

The present invention is preferably used in flat sheet form, but it is suitable for use in the corrugated (pleated) form in a filter element so as to provide a large membrane surface area for the volume of the filter element. In this format, a capsule-type housing, such as the housing currently used with the Opticap^{™} filter sold by Millipore Corporation. The filter element can comprise a single filtration medium of the present invention or can comprise multiple such filtration media adhered together. The other aspects of the filter element may be of any suitable construction and prepared from any suitable material. The filter element can be constructed using techniques that are well known in the art.

In a preferred embodiment of the present invention, the layers of the filtration material are substantially similar. Preferably, the membranes are comprised of polyethersulfone or regenerated cellulose. Another preferred embodiment is when the filtration material is a composite membrane. Those of ordinary skill in the art can select other materials that are suitable for selecting or producing the filtration material of the present invention.

In a preferred embodiment, the upstream layer of the filtration material is a high integrity ultrafiltration membrane. Preferably, all membrane layers of the filtration material are high integrity ultrafiltration membranes.

In the present invention the device has filtration material that includes three layers and the layers are oriented TSDS.

In a preferred embodiment, the device of the present invention has a housing suitable for receipt of liquid from a syringe.

In another preferred embodiment, the device of the present invention is adapted for dead-ended filtration. Preferably, the housing of this device is a capsule. In another embodiment of the present invention, the housing is adapted to receive the filtration material in disk form and may be reused.

The following examples further illustrate the present invention.

### Example 1

The following example concerns an ultrafiltration polymeric membrane suitable for the multi-layered virus removal material of the present invention. It is an example of a high integrity ultrafiltration membrane.

An ultrafiltration membrane was made by dissolving 17-22% by weight of Rade1 A 200, a polyethersulfone manufactured by Amoco Chemicals of Alpharetta, GA, in a mixture of triethylene glycol ("TEG") and N-methylpyrrolidone ("NMP") in a ratio of TEG/NMP of 1.8, the solution was then filtered and degassed. Note that the expected cloud point of the solution upon heating occurs at 50°C. The solution was then cast onto a 0.0381 mm (0.0015 inch) thick Mylar belt passing over a casting drum submerged in water kept at 57°C, using a knife to set the cast thickness to about 200 µm, with only about 12.7 mm (1/2 inch) of cast film exposed to dry air before the point of immersion in the water bath. The casting speed was 4.572 to 9.144 m/min (15 to 30 ft/min).

The formed membrane is extracted by passing through additional water baths and dried using an impingement drier with air temperature controlled at about 70°C. The membrane is then hydrophilized by passing it through a solution of Sartomer 9035. The hydrophilization procedure followed was the one disclosed by U.S. Patent No. 4,618,533.

The Sartomer 9035 is an alkoxylated water-soluble triacrylate manufactured by Sartomer Chemical Co. of West Chester, PA. The monomer solution includes the monomer dissolved in a mixture of Igracure 2959, acetone, hexylene glycol (4-methyl -2,4-pentanediol MPD), and water in the following weight %:
[ 3.0/0.25/2.0/25.0/69.75 ], respectively. Igracure 2959 is 4-(2-Hydroxyethoxy)phenyl-(2-hydroxy-2-propyl)-ketone and is available from Ciba Additives of Tarrytown, NY.

The monomer solution fully wet the membrane, which was then exposed to UV radiation in order to polymerize and cross-link a hydrophilic coating on the surfaces of the membrane. After additional extraction in water and drying, an ultrafiltration membrane is recovered which typically exhibits a water permeability of 7.253-29.011 lmh/kPa (50-200 lmh/psi) when tested in one layer.

The membrane preferably had an isopropyl alcohol (IPA) automated ramp bubble point (AMP) greater than 613.63 kPa (89 psi) when tested in TSDS orientation with a porometer manufactured by PMI of Ithaca, NY. For the ABP test method, the BP is registered at a sudden transition between low level "random, diffusion" bubbles that occur below the BP and a burst of bubbles that occur at the BP. This transition was very clear for the present invention

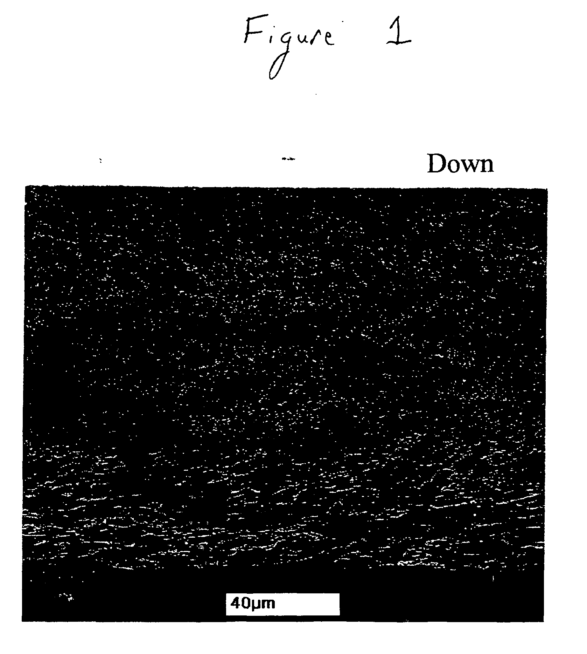

Figure 1 provides a SEM of this membrane. It shows a tight side ultrafiltration membrane that is a high integrity ultrafiltration membrane as defined above. It is to be used at least as the upstream layer, but of course, it is preferable to use this membrane in all the layers of the filtration material of the present invention. For virus removal in accordance with the present invention, the upstream layer is in the TSDS orientation, with the following layer(s) that are in TSDS orientation. The membrane configuration includes 3 layers (TSDS/TSDS/TSDS) or more layers in similar arrangements where the nomenclature in parentheses refers to orientation of individual layers with the upstream layer listed first.

As an example, a 3-layer configuration (TSDS/TSDS/TSDS) of a membrane prepared as described above was shown to fully retain viruses as demonstrated by absolute removal of bacteriophage φ6 (size 78 nm) when challenging with a suspension of at least 10 pfu/ml up to volumes of 50 ml/cm² of tested filter. There was no virus detected downstream of the 3-layer filter both in buffer only as well as in the presence of monoclonal IgG at a concentration 2.5 g/ml. IgG passage was > 98%. Average processing flux in presence of IgG was about 2.9 lmh/kPa (20 lmh/psi) averaged over the full volume filtered (∼20 ml/cm²).

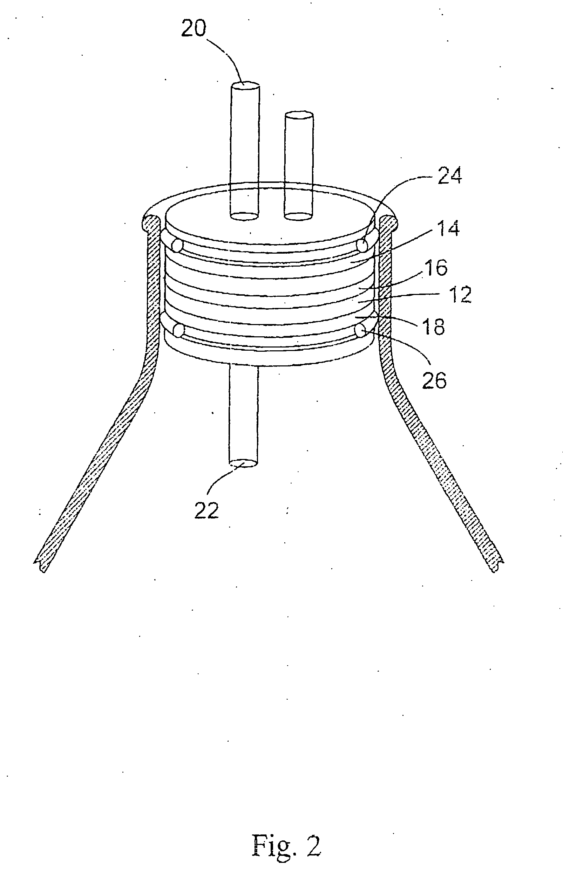

The layers were in 47 mm disk form and were stacked in a housing represented by Figure 2. No gluing or bonding of the layers of the present invention is necessary. The periphery of the filtration material must be secured, however, to insure fluid only flows through the membrane. The housing 12 contained the upstream layer 14, intermediate layer 16 and the third or downstream layer 18. To insure the fluid flowed in the inlet 20, through the membranes and through the outlet 22, two o-rings, 24 and 26, were employed in the housing.

This example represents large virus removal by the present invention and demonstrates substantial removal of large viruses is possible at a high processing flux with substantial passage of relatively large proteins such as IgG. A comparable product available on the market, DV50, achieves a low flux, only 0.29 lmh/kPa (2 lmh/psi) under similar conditions. The present invention is directed to large virus removal with high process fluxes, that is, > 0.725 lmh/kPa (5 lmh/psi). Preferably, the process flux is > 1.45 lmh/kPa (10 lmh/psi). More preferably, the process flux is > 2.901 lmh/kPa (20 lmh/psi).

### Example 2

The membrane described in Example 1 was fabricated into pleated cartridges. Full removal of φ6 virus was demonstrated when a pleated cartridge made with three layers (orientation TSDS/TSDS/TSDS) of the membrane prepared as described in the above example was challenged with a virus suspension of about 10⁷ pfu/ml. The cartridges each had an effective surface area of 4900 cm² and 4 liters of virus suspension were used as a challenge solution. No virus was detected downstream of the three cartridges that were tested. The average processing flux was about 2.61 lmh/kPa (18 lmh/psi), in substantial agreement with the flux observed in testing flat stock membrane.

### Example 3

A regenerated cellulose composite membrane (sold as the Ultracell^{™} membrane by Millipore Corporation and found as catalog No. PLCXK) rated as 300 kDa nominal molecular weight was used in a virus retention test in 1, 2 and 3 layers in TSDS, TSDS/TSDS, and TSDS/TSDS/TSDS orientation, respectively. A model bacteriophage (size 28 nm) φX174 was used in Sorensen's buffer at a challenge level of about 10 pfu/ml. Filtration was done at 68.947 kPa (10 psi) and 250 ml of filtrate was collected from each 47 mm holder (See Figure 2). Flux and LRV was measured. Flux was only slightly lower than the water flux values estimated from known water permeability for this membrane. Table 1 provides the results of testing the regenerated cellulose membrane with a number of layers.

**Table 1**

| **Layers** | **Orientation** | **LRV of φX174** | **Flux-lmh/kPa(lmh/psi)** |
|---|---|---|---|
| 1 | TSDS | 4.9 | 23.21 (160) |
| 2 | TSDS/TSDS | >6.9 | 9.28 (64) |
| 3 | TSDS/TSDS/TSDS | >6.9 | 6.96 (48) |

### Example 4

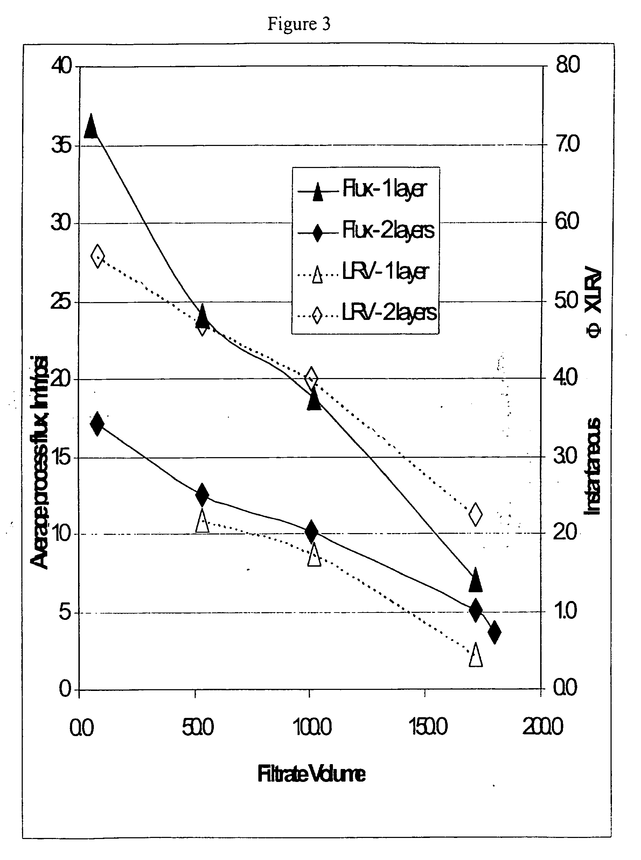

A 25 mg/ml solution of monoclonal antibody (MAb, molar mass approx. 160 kDa) in Tris-HCl buffer was spiked with model bacteriophage (size 28 nm) FX174 at a challenge level about 10⁷ pfu/ml. The solution was filtered at 206.84 kPa (30 psi) through 47 mm disks of composite ultrafiltration membrane manufactured according to U.S. Patent No. 5,017,292 and marketed under the trade name Viresolve^{™} by Millipore Corporation. A side-by-side comparison was done. One holder contained 1 membrane layer in TSDS orientation; another holder contained 2 membrane layers in TSDS/TSDS orientation. Filtrate volume, time, concentration of virus and monoclonal antibody in filtrate was measured several times during the filtration. Flux, virus LRV and MAb passage were calculated. Figure 3 shows the resulting values of flux and virus LRV. The MAb passage (not shown in Figure 3) was >98% for all values measured at points corresponding to those in Figure 3.

This example represents small virus removal by the present invention and demonstrates substantial removal of small viruses is possible at a high processing flux with substantial passage of relatively large proteins such as IgG. A comparable product available on the market, DV20, achieves a low flux, only 0.6 lmh/psi under similar conditions. The present invention is directed to small virus removal with high process fluxes, that is, > 0.29 lmh/kPa (2 lmh/psi). Preferably, the process flux is > 0.43 lmh/kPa (3 lmh/psi). More preferably, the process flux is > 0.58 lmh/kPa (4 lmh/psi).

## Claims

1. A device for conducting high flux removal of a virus from a protein-containing solution, the device comprising:
a housing (12) having an inlet (20) for receiving the solution to be filtered and an outlet (22) for removing filtrate, and containing three asymmetric membranes (14,16,18) stacked on top of each other, wherein
said asymmetric membranes (14,16,18) are each hydrophilized and each have a tight-side and an open-side, the average pore size of said tight-side being smaller than the average pore size of said open-side,
said asymmetric membranes (14,16,18) are orientated with their tight side downstream (TSDS),
the flux is greater than 0.29 lmh/kPa (2 lmh/psi), and
said asymmetric membrane have an IPA AMP of at least 613.63 kPa (89 psi).

2. The device of claim 1, wherein said asymmetric membranes are pleated.

3. The device of claim 1, wherein said asymmetric membranes are defined to have a log reduction value (LRV) greater than 6 and a protein passage greater than 98% when challenged with a suspension of at least 10 pfu/ml of bacteriophage size 78nm and monoclonal IgG at a concentration 2.5 mg/ml up to volumes of 50 ml/cm².

4. The device of any one of claims 1 to 3, where in the housing (12) is suitable for receipt of liquid from a syringe.

5. The device of any one of claims 1 to 4, where in the housing is a capsule.

6. The device of any one of claims 1 to 5, wherein the housing (12) is adapted to receive the filtration material in disk form and is reusable.

7. The device of any one of claims 1 to 5, wherein the housing is adapted to receive the filtration material in cartridge form and is reusable.

8. A method for removing virus from a protein-containing solution at a high flux, said method comprising filtering the protein-containing solution with the device of any one of claims 1 to 7.

9. The method of claim 8, wherein the high flux is greater than 0.29 lmh/kPa (2 lmh/psi).

## Patentansprüche

1. Vorrichtung zur Entfernung eines Virus aus einer Protein enthaltenden Lösung bei hohen Durchflussmengen, wobei die Vorrichtung umfasst:
ein Gehäuse (12), das einen Einlaß (20) zum Aufnehmen der zu filternden Lösung und einen Auslaß (22) zum Entfernen von Filtrat hat und drei asymmetrische Membranen (14,16,18) enthält, die aufeinandergeschichtet sind, wobei
die asymmetrischen Membranen (14,16,18) jeweils hydrophilisiert sind und jeweils eine dichte Seite und eine offene Seite aufweisen, wobei die durchschnittliche Porengröße der dichten Seite geringer ist als die durchschnittliche Porengröße der offenen Seite,
wobei die asymmetrischen Membranen (14,16,18) mit ihrer dichten Seite stromabwärts (TSDS) ausgerichtet sind,
wobei die Durchflussmenge größer ist als 0,29 lmh/kPa (2 lmh/psi), und
wobei die asymmetrischen Membranen ein IPA AMP von mindestens 613,63 kPa (89) psi aufweisen.

2. Vorrichtung nach Anspruch 1, wobei die asymmetrischen Membranen gefaltet sind.

3. Vorrichtung nach Anspruch 1, wobei die asymmetrischen Membranen einen log-Reduktionswert ("log reduction value" - LRV) größer als 6 und einen Proteindurchgang größer als 98% aufweisen, wenn sie mit einer Suspension von mindestens 10 pfu/ml einer Bakteriophage der Größe 78 nm und monoklonalem IgG mit einer Konzentration von 2,5 mg/ml bis zu Volumen von 50 ml/cm² beaufschlagt werden.

4. Vorrichtung nach einem der Ansprüche 1 bis 3, wobei das Gehäuse (12) zur Aufnahme einer Flüssigkeit aus einer Spritze geeignet ist.

5. Vorrichtung nach einem der Ansprüche 1 bis 4, wobei das Gehäuse eine Kapsel ist.

6. Vorrichtung nach einem der Ansprüche 1 bis 5, wobei das Gehäuse (12) das Filtrationsmaterial in Scheibenform aufnehmen kann und wiederverwendbar ist.

7. Vorrichtung nach einem der Ansprüche 1 bis 5, wobei das Gehäuse das Filtrationsmaterial in Kartuschenform aufnehmen kann und wiederverwendbar ist.

8. Verfahren zum Entfernen von Virus aus einer Protein enthaltenden Lösung bei hoher Durchflussmenge, wobei das Verfahren das Filtern der ein Protein enthaltenden Lösung mit der Vorrichtung nach einem der Ansprüche 1 bis 7 umfaßt.

9. Verfahren nach Anspruch 8, wobei die hohe Durchflussmenge größer als 0,29 lmh/kPa (2 lmh/psi) ist.

## Revendications

1. Dispositif pour l'élimination à haut flux d'un virus d'une solution contenant des protéines, dispositif comprenant :
un boîtier (12) comprenant un orifice d'admission (20) pour recevoir la solution à filtrer et un orifice de sortie (22) pour évacuer le filtrat, et contenant trois membranes asymétriques (14, 16, 18) empilées les unes sur les autres, dans lequel
lesdites membranes asymétriques (14, 16, 18) sont chacune rendues hydrophiles et ont chacune une face étanche et une face libre, le diamètre moyen des pores de ladite face étanche étant inférieur au diamètre moyen des pores de ladite face libre,
lesdites membranes asymétriques (14, 16, 18) étant orientées avec leur face étanche vers l'aval (FEVA),
le flux est supérieur à 0,29 lmh/kPa (2 lmh/psi), et
lesdites membranes asymétriques ont un AMP IPA d'au moins 613,63 kPa (89 psi).

2. Dispositif suivant la revendication 1, dans lequel lesdites membranes asymétriques sont plissées.

3. Dispositif suivant la revendication 1, dans lequel lesdites membranes asymétriques sont définies de manière à avoir une valeur logarithmique de réduction (LRV) supérieure à 6 et un passage des protéines supérieur à 98 % lors de l'épreuve d'administration d'une suspension d'au moins 10 ufp/ml d'un bactériophage ayant des dimensions de 78 nm et d'une IgG monoclonale à une concentration de 2,5 mg/ml jusqu'à des volumes de 50 ml/cm².

4. Dispositif suivant l'une quelconque des revendications 1 à 3, dans lequel le boîtier (12) est apte à recevoir un liquide provenant d'une seringue.

5. Dispositif suivant l'une quelconque des revendications 1 à 4, dans lequel le boîtier est une capsule.

6. Dispositif suivant l'une quelconque des revendications 1 à 5, dans lequel le boîtier (12) est adapté à recevoir la matière de filtration sous forme de disque et est réutilisable.

7. Dispositif suivant l'une quelconque des revendications 1 à 5, dans lequel le boîtier est adapté à recevoir la matière de filtration sous forme de cartouche et est réutilisable.

8. Procédé pour éliminer un virus d'une solution contenant des protéines, à un flux élevé, ledit procédé comprenant la filtration de la solution contenant des protéines avec le dispositif de l'une quelconque des revendications 1 à 7.

9. Procédé suivant la revendication 8, dans lequel le flux élevé est supérieur à 0,29 lmh/kPa (2 lmh/psi).
